(19) Europäisches Patentamt
European Patent Office
Office européen des brevets

(11) Publication number: **0 323 590 A2**

# (12) EUROPEAN PATENT APPLICATION

(21) Application number: **88120926.6**

(22) Date of filing: **14.12.88**

(51) Int. Cl.4: **C07C 109/06 , C07C 133/02 , C07D 213/79 , A61K 31/175**

Claims for the following Contracting States: ES + GR.

(30) Priority: **24.12.87 JP 325436/87**

(43) Date of publication of application:
**12.07.89 Bulletin 89/28**

(84) Designated Contracting States:
**AT BE CH DE ES FR GB GR IT LI LU NL SE**

(71) Applicant: **ONO PHARMACEUTICAL CO., LTD.**
**No. 14, Doshomachi 2-chome Higashi-ku**
**Osaka-shi Osaka(JP)**

(72) Inventor: **Ohuchida, Shuichi Ono Pharmaceutical Co., Ltd.**
**Minase Res. Institute 3-1-1 Sakurai**
**Shimamoto-cho**
**Mishima-gun Osaka(JP)**
Inventor: **Toda, Masaaki Ono Pharmaceutical Co., Ltd.**
**Minase Res. Institute 3-1-1 Sakurai**
**Shimamoto-cho**
**Mishima-gun Osaka(JP)**
Inventor: **Miyamoto, Tsumoru Ono Pharmaceutical Co., Ltd.**
**Minase Res. Institute 3-1-1 Sakurai**
**Shimamoto-cho**
**Mishima-gun Osaka(JP)**

(74) Representative: **Henkel, Feiler, Hänzel & Partner**
**Möhlstrasse 37**
**D-8000 München 80(DE)**

## (54) Carbazoyl derivatives.

(57) A carbazoyl derivative of the general formula:

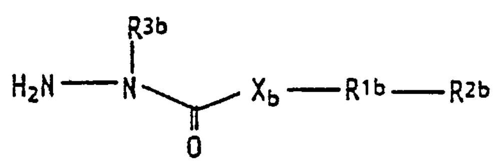

(IB)

(wherein, $R^{1b}$ represents a bond, alkylene group of from 1 to 6 carbon atom(s) or alkenylene group of from 2 to 6 carbon atoms,
$R^{2b}$ represents a carbocyclic or heterocyclic ring unsubstituted or substituted by from one to three halogen atom(s), hydroxy group, nitro group, amino group, alkyl or alkoxy group of from 1 to 4 carbon atom(s), 3-aminoureido group, phenoxy group or acylamino group of from 2 to 5 carbon atoms, or $R^{1b}$ together with $R^{2b}$, represents an alkyl group of from 1 to 12 carbon atom(s) unsubstituted or substituted by 3-aminoureido group,
$R^{3b}$ represents (1) a hydrogen atom,
(2) an alkyl group of from 1 to 6 carbon atom(s) or
(3) a phenyl or benzyl group unsubstituted or substituted by from one to three halogen atom, alkyl or alkoxy group of from 1 to 4 carbon atom(s), hydroxy group or nitro group,
$X_b$ represents a bond or imino gorup,
with the proviso that the compounds wherein the groups shown by $X_b$-$R^{1b}$-$R^{2b}$ represent a phenyl group, 4-

EP 0 323 590 A2

aminophenyl gorup, anilino group and 2-thienyl group and the groups shown by $R^{3b}$ represent a hydrogen atom, are excluded.),
or an acid addition salt thereof possesses inhibitory activity on Maillard reaction, and therfore is useful for treating and/or prevention of several diabetic complications and deseases induced by aging.

## Carbazoyl Derivatives

Summary

This invention is related to carbazoyl derivatives and medicines containing them as active ingredients. More particularly, this invention is related to

(1) novel carbazoyl derivatives of the general formula

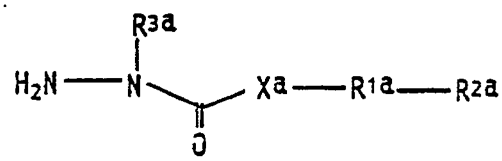

(IA)

(wherein, all of the symbols represent the same meaning as hereinafter defined.),

(2) process for their preparation and

(3) inhibitory agents on Maillard reaction containing the compound of the general formula:

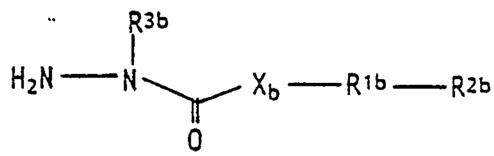

(IB)

(wherein, all of the symbols represent the same meaning as hereinafter defined.), which includes the compounds of the fomula (IA), as active ingredient.

[Background]

In 1912, Maillard reported that, when a mixture of amino acid and reducing sugar is heated, it shows brown color (called browning phenomenon).
[maillard, L.C., Compt. Rend. Soc. Biol., 72, 599 (1912)], and suggested that this reaction might occur in a body.

In 1968, Rahbar reported that HbAlc, the glycosylated part of haemoglobin (Hb), was increased in diabetics [Rahbar, S., Clin. Chim. Acta., 22, 296 (1968)].

Afterwards, it became clean that HbAlc had the chemical structure in which glucose binds to valine in N-terminal side of $\beta$-chain in the form of Amadori rearrangement [Koenig, R.J., Blobstein, S.H., & Cerami, A., J. Biol. Chem., 252, 2992 (1977)], and that Maillard reaction proceeds nonenzymatically [Stevens, V.J., Vlassara, H., Abati, A., & Cerami, A., J. Biol. Chem., 252, 2998 (1977)]. These results showed that Maillard reaction occurs in a body.

As an initial step, Maillard reaction consists of forming the Amadori rearrangement products by glycosylation of reducing sugar with amino-group of protein. In the advanced stage of this reaction

① cross-linked compounds [called advanced glycosylation products (abbreviated as "AGE")] are produced,

② solubility of them becomes low,

③ the products can not be easily degraded by the action of proteases,

④ a fluorescent is formed, and then

⑤) the products are colored brown.

The mechanism of AGE production has been proposed by some groups. For example, the theory of Brownlee et al is shown below [Brownlee, M. et al., Science, 232, 1629 (.1986)].

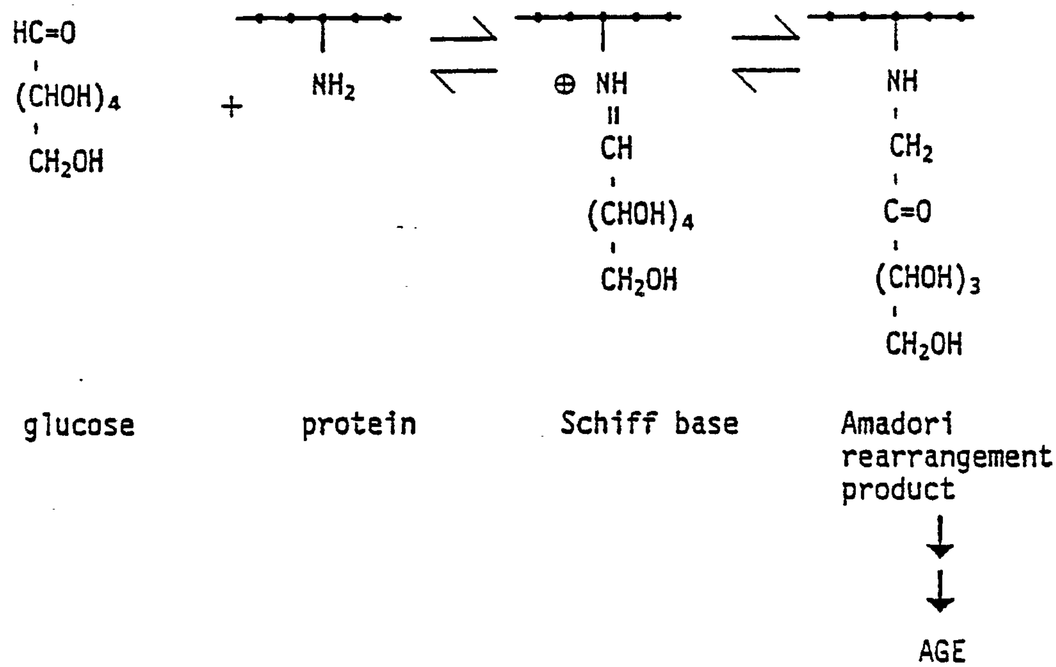

glucose protein Schiff base Amadori rearrangement product AGE

Maillard reaction is observed in healthy person, and particulary this is caused notably in diabetics that a level of blood sugar is high and in protein of which metabolic turnover is slow.

For example, in the case of haemoglobin, the level of glycosylation in diabetic mouse are 2.7 times as high as that of normal mouse [Monnier, V.M. et al., The Maillard Reaction in Foods and Nutrition, ACS Symposium Series, 215, 432, Am. Chem. Soc., Washington, D.C. (1983)], and glycosylation of serum albumin in diabetics was advanced [Guthrow, C.E. et al., Proc. Natl. Acad. Sci. U.S. 76, 4258 (1979)].

Further, it was turned out that, when a glycosylated serum protein was administrated intravenously during 12 weeks, the typical diabetic renal lesion was observed [Monnier, V.M. et al., Clin. Endocrinol. Metab., 11, 431 (1982)].

Crystallin in lens is the special protein which is not metabolized at all after its biosynthesis. When crystallin was glycosylated, its steric structure was transformed and the SH groups were oxidized to form S-S bond. A sequence of processes induced to polymerize the protein in crystallin.

In the case of diabetic cataract in rats, the ratio of binding of protein and glucose was ten times as high as that of normal rat, and also intramolecular S-S bond formation increased [Monnier, V.M. & Cerami, A. Clin. Endocrinol. Metab. 11, 431 (1982)].

Glycosylation of crystallin caused its polymerization, decrease in its solubility, formation of fluorescent substance, yellow and brown coloring. This phenomenon is very similar to that of lens by aging [Chiou, S.H., Chylack, L. T., Jr., Tung, W.H., & Bunu, F., J. Biol. Chem. 256, 5176 (1981)].

Collagen and elastin in connective tissue contain lysine and hydroxylysine abundantly, the speed of their metabolic turnover is slow, and the existance of reactants with glucose at the basement membrance of renal adrenal glands, skin and tendon has been found [Monnier, V.M., Stevens, V.J., & Cerami, A., Maillard Reactions in Food, Prog. Food Nutr. Sci. 5, 315, Pergamon Press, London], further these glycosylation products might be related to sclerosis in a blood vessel wall [Rosenburg, H., Modrak, J.B., Hassing, J.M., Al-Turk, W.A., & Stohs, S.J., Biochem.Biophys.Res. Commun, 91, 498 (1979)].

The cause of diabetic neurosis may be nonenzymatic glycosylation of neuro-myelin protein [Monnier, V.M. et al., Clin.Endocrinol.Metab. 11, 431 (1982)].

In this way, Maillard reaction in a body is related to not only various diabetic complication but also a lot of diseases accompanied with aging.

[Prior arts]

On the above background, recently, researches of Maillard reaction inhibitors has been carried out. For example, Brownlee, M. et al. showed that aminoguanidine inhibits Maillard reaction in vitro, and that aminoguanidine, administered to diabetic rats, inhibits diabetes-induced accumulation of advanced glycosylation end products in arterial wall connective tissue protein [Brownlee, M. et al., Science, 232, 1629

(1986)].

They have considered that the amino group of a nucleophilic hydrazine compound (i.e. the amino group which is bonded to guanidino group in aminoguanidine) blocks reactive carbonyls on early glycosylation products, and inhibits further cross-link formation of chemically reversible Amadori product.

Further, in the specification of Japanese Patent Kokai No. 62-142114 i.e. European Patent Publication No. 222313, it was suggested that the pharmaceutical composition comprising compound having the group containing reactive nitrogen atoms, which enable to react with reactive carbonyls in chemically reversible Amadori product, inhibits the production of advanced glycosylation end products. Concretely, the compositions comprising aminoguanidine, α-hydrazino histidine and lysine are disclosed.

And recently, urea, guanidine and salts thereof were disclosed concretely as compositions which inhibit cross-link formation of collagen in the specification of Japanese Patent Kokai No. 62-249908 i.e WPI Accession No.87-345514/49.

The known compounds in the compounds of the present invention are isoniazid (is known to possess an antibiotic action), acetohydrazide, benzoylhydrazine, 4-aminobenzoylhydrazine, 2-nitrobenzoylhydrazine, 4-nitrobenzoylhydrazine, 2-chlorobenzoylhydrazine, 4-chlorobenzoylhydrazine, benzylhydrazide, 3-bromobenzoylhydrazine, 4-bromobenzoylhydrazine, 2-thiophencarboxylic acid hydrazide, 4-phenylsemicarbazide and p-hydroxybenzoylhydrazine. The compounds except for isoniazid are not known to possess pharmacological actions.

[Disclosure of the invention]

The present invention is related to novel carbazoyl derivatives of the general formula:

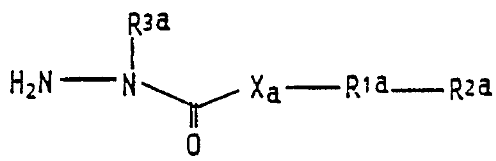

(IA)

(wherein, $R^{1a}$ represents a bond, alkylene group of from 1 to 6 carbon atom(s) or alkenylene group of from 2 to 6 carbon atoms,
$R^{2a}$ represents a carbocyclic or heterocyclic ring unsubstituted or substituted by from one to three halogen atom(s), hydroxy group(s), nitro group(s), amino group(s), alkyl or alkoxyl group(s) of from 1 to 4 carbon atom(s), 3-aminoureido group(s), phenoxy group(s) or acylamino group(s) of from 2 to 5 carbon atoms, or
$R^{1a}$ together with $R^{2a}$, represents an alkyl group of from 1 to 12 carbon atom(s) unsubstituted or substituted by 3-aminoureido group,
$R^{3a}$ represents (1) a hydrogen atom,
(2) an alkyl group of from 1 to 6 carbon atom(s) or
(3) a phenyl or benzyl group unsubstituted or substituted by from one to three halogen atom(s), alkyl or alkoxy group(s) of from 1 to 4 carbon atom(s), hydroxy group(s) or nitro group(s),
$X_a$ represents a bond or imino group;
with the proviso that, the compounds in which the group shown by $-X_a-R^{1a}-R^{2a}$ Rrepresents methyl group, phenyl group, 4-aminophenyl group, anilino group, 2-nitrophenyl group, 4-nitrophenyl group, 2-chlorophenyl group, 4-chlorophenyl group, benzyl group, 3-bromophenyl group, 4-bromophenyl group, 2-thienyl group, 4-pyridyl group and 4-hydroxyphenyl group, and $R^{3a}$ represents hydrogen atom, are excluded.) and acid addition salts thereof,
2) precess for their preparation and
3) inhibitory agents on Maillard reaction containing the compound of the general formula:

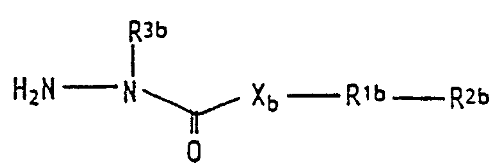

(IB)

(wherein, $R^{1b}$ represents a bond, alkylene group of from 1 to 6 carbon atom(s) or alkenylene group of from 2 to 6 carbon atoms,
$R^{2b}$ represetns a carbocyclic or heterocyclic ring unsubstituted or substituted by from one to three halogen atom(s), hydroxy gorup(s), nitro group(s), amino group(s), alkyl or alkoxy group of from 1 to 4 carbon atom-(s), 3-aminoureido group, phenoxy group or acylamino group of from 2 to 5 carbon atom(s), or
$R^{1b}$ together with $R^{2b}$, represents an alkyl group of from 1 to 12 carbon atom(s) unsubstituted or substituted by 3-aminoureido group,
$R^{3b}$ represents (1) a hydrogen atom,
(2) an alkyl group of from 1 to 6 carbon atom(s) or
(3) a phenyl or benzyl group unsubstituted or substituted by from one to three halogen atom(s), alkyl or alkoxy group(s) of from 1 to 4 carbon atom(s), hydroxy group(s) or nitro group(s),
$X_b$ represents a bond or imino group;
with the proviso that, the compounds in which $-X_b-R^{1b}-R^{2b}$ represents a phenyl group, p-aminophenyl group, anilino group or 2-thienyl group, and $R^{3b}$ represents a hydrogen atom.) or acid addition salts thereof as active ingredients.

The terms of alkyl group, alkylene group, alkenylene group and alkoxy group in description each symbol throughout the present specification including claims mean straight-chain or branched-chain alkyl group, alkylene group, alkenylene group and alkoxy group.

In the general formula (IA) and (IB), alkylene groups of from 1 to 6 carbon atom(s) shown by $R^{1a}$ and $R^{1b}$ are methylene, ethylene, trimethylene, tetramethylene, pentamethylene, hexamethylene group and isomeric groups thereof. Alkenylene groups of from 2 to 6 carbon atoms are vinylene, propenylene, butenylene, pentenylene, hexenylene group and isomeric groups thereof. All groups are preferable. $R^{1a}$ and $R^{1b}$ are also preferable to represent bonds. Especially preferable $R^{1a}$ and $R^{1b}$ are bond, methylene, ethylene trimethylene, vinylene and 1-pentenylene group.

In the general formula (IA) and (IB), halogen atoms shown as substituent groups in $R^{2a}$, $R^{2b}$, $R^{3a}$ and $R^{3b}$ are fluorine, chlorine, bromine and iodine atom. Alkyl group of 1 to 4 carbon atom(s) are methyl, ethyl, propyl, butyl group and isomeric group thereof. Alkoxy groups of from 1 to 4 carbon atom(s) are methoxy, ethoxy, propoxy, butoxy group and isomeric groups thereof. Acylamino groups of from 2 to 5 carbon atoms shown as substituent groups in $R^{2a}$ and $R^{2b}$ are acetylamino, propionylamino, butyrylamino, valerylamino group and isomeric groups thereof. All groups are preferable. Substituent groups in $R^{2a}$ and $R^{2b}$ are also preferable to represent hydroxy group, nitro group, amino group, 3-aminoureido and phenoxy group. Substituent groups in $R^{3a}$ and $R^{3b}$ are preferable to represent hydroxy group and nitro group. Especially preferable substituent groups in $R^{2a}$ and $R^{2b}$ are fluorine atom, chlorine atom, methyl group, methoxy group, nitro group, amino group and 3-aminoureido group. $R^{2a}$ and $R^{2b}$ are also preferable to represent unsubstituted carbocyclic and heterocyclic ring. $R^{3a}$ and $R^{3b}$ are also preferable to represent unsubstituted phenyl group an benzyl group. In the general formula (IA) and (IB), carbocyclic rings in $R^{2a}$ and $R^{2b}$ are mono-, bi- or tri-aromatic carbocyclic rings containg not more than 15 carbon atoms which may be partially or fully saturated.

The rings are, for example, benzene, naphthalene, indene, aqulene, fluorene, phenanthrene, anthracene, acenaphthylene, biphenylene ring and the rings which may be partially or fully saturated thereof. Preferable rings are benzene, naphthalene, cyclohexane and cyclopentane.

In the general formula (IA) and (IB), heretocyclic rings in $R^{2a}$ and $R^{2b}$ are mono-, bi- or tri- aromatic heterocyclic rings containing not more than 15 carbon and hetero atoms which may be partially or fully saturated. For example, they are furan, thiophene, pyrrole, oxazole, isooxazole, thiazole, isothiazole, imidazole, pyrazole, furazan, pyran, pyridine, pyridazine, pyrimidine, pyrozine, indole, isoindole, benzofuran, benzothiophene, indolizine, chromene, quinoline, isoquinoline, quinolizine, purine, indazole, quinazoline, cinnoline, quinoxaline, phthalazine, pteridine, carbazole, acridine, phenanthridine, xanthene, phenazine, phenothiazine rings, and partially or fully saturated rings thereof. Pyridine ring is preferable.

Alkyl groups of from 1 to 12 carbon atom(s) shown by $R^{1a}$ together with $R^{2a}$ are methyl, ethyl, propyl, butyl, pentyl, hexyl, heptyl, octyl, nonyl, decyl, undecyl, dodecyl group and isomeric groups thereof. All groups are preferable. These groups are also preferable to be substituted by 3-aminoureido group. Especially preferable groups are isopropyl, butyl, octyl, nonyl and 6-(3-aminoureido)hexyl group.

In the general fromula (IA) and (IB), alkyl groups of from 1 to 6 carbon atom(s) shown by $R^{3a}$ and $R^{3b}$ are methyl, ethyl, propyl, butyl, pentyl, hexyl group and isomeric gorups thereof. All groups are preferable. $R^{3a}$ and $R^{3b}$ are preferable to represent a hydrogen atom. Especially preferable $R^{3a}$ and $R^{3b}$ are a hydrogen atom, methyl, propyl and benzyl group. $X_a$ and $X_b$ represent a bond and imino group and both groups are preferable.

Throughout the specification, stereoisomers generated by stereo configurations (asymmetric carbon,

double bond etc.) and structural isomers generated by branch of carbon chain etc. are included in the present invention.

And the compounds of the general formula (IA) and (IB), if desired, may be converted into acid addition salts by the known methods.

Preferably, acid addition salts are non-toxic salts and water-soluble.

The suitable acid addition salts are, for example, hydrochloric acid, hydrobromic acid, hydroiodic acid, sulfuric acid, phosphoric acid, an inorganic acid such as nitric acid, or an organic acid such as acetic acid, lactic acid, tartric acid, benzoic acid, citric acid, methanesulphonic acid, ethanesulphonic acid, benzenesulphonic acid, toluenesulphonic acid, isethionic acid, glucuronic acid and gluconic acid.

Acid addition salts may be obtained by the known methods, for example, by reacting stoichiometric quantities of a compound of general formula (IA) and (IB) and the appropriate acid in a suitable solvent.

[Comparison with prior arts]

The compounds of the present invention are carbozoyl derivatives. Accordingly, the compounds are significantly different from the prior arts i.e. aminoguanidine, α-hydrazinohystidine, lysine and guanidine, in chemical structure. It can be unexpected that the compounds which have significantly different structures also possess same pharmacological action. About the known compounds among the compounds of the present invention, their pharmacological effect are not known except for antibiotic effect of isoniazid. Accordingly it can be unexpected that the compounds of the present invention possess an inhibitory action on Maillared reaction over drug efficacies of these prior arts. Moreover, among the compounds of the present invention, the compounds having similar structures to urea whose inhibitory action on Maillard reaction have been already known are confirmed to possess significantly stronger inhibitory action than urea. It can be unexpected that the introduction of substituents leads to increase of activity.

[Process for the preparation of the compounds of the present invention]

The compounds of the general formula (IA) may be prepared by the step described hereinafter.

Step 1:

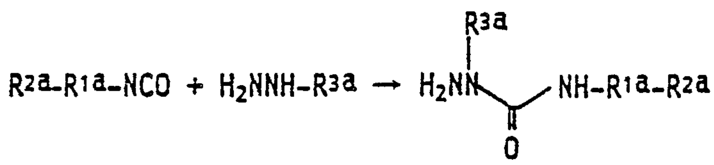

(II) (III) (IA-1)

Step 2:

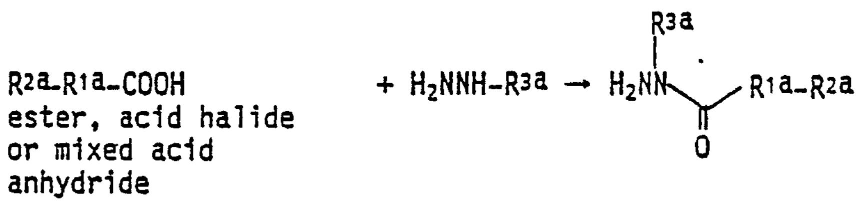


(IV) (III) (IA-2)

Step 3:

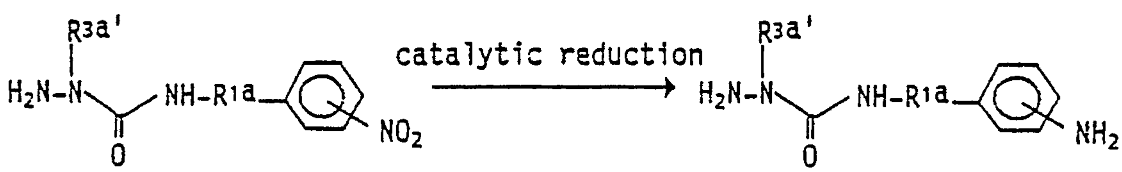


(IA-3) (IA-4)

Step 4:

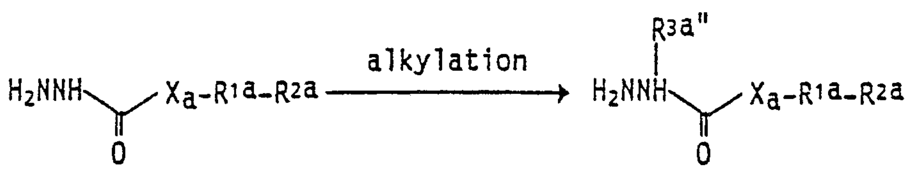


(IA-5) (IA-6)

(wherein, $R^{3a'}$ represents 1) a hydrogen atom, 2) an alkyl group of from 1 to 6 carbon atom(s) or 3) a phenyl or benzyl group unsubstituted or substituted by from one to three halogen atom(s), alkyl or alkoxy group(s) of from 1 to 4 carbon atom(s) or hydroxy group(s), $R^{3a''}$ represents 1) an alkyl group of from 1 to 6 carbon atom(s) or 2) a phenyl or benzyl group unsubstituted or substituted by from one to three halogen atom(s) alkyl or alkoxy gorup(s) of from 1 to 4 carbon atom(s), hydroxy group(s) or nitro group(s), and the other symbols represent the same meaning as hereinafter defined.)

Step 1 may be carried out by reacting the isocyanate derivative of the general formula (II) and the compound of the general formula (III) in an inert organic solvent (diethyl eter etc.) at a temperature of from 0°C to 40°C.

Step 2 may be carried out by ① reacting the ester derived from the carboxylic acid of the general formula (IV) and the compound of the general formula (III) in an inert organic solvent (methanol, ethanol etc) at a temperature of from 0°C to 40°C, ② reacting the acid halide derived from the carboxylic acid of the general formula (IV) and the compound of the general formula (III) in the presence or absence of a base, for example a tertiary amine (triethylamine, pyridine, picoline etc.) in an inert organic solvent (tetrahydrofuran

etc.) at a temperature of from -5° C to 40° C, or ③ reacting the mixed acid anhydride derived from the compound of the general formula (IV) and the compound of the general formula (III) in an inert organic solvent (diethyleter, tetrahydrofuran etc.) at a temperature of from 0° C to 40° C. Ester described herein is, for example, methyl ester, ethyl ester, and acid halide is, for example, an acid chloride. Mixed acid chloride described herein are, for example, acid anhydrides of the carboxylic acid of the general formula (IV) and ethyl chloroformate or isobutyl chloroformate.

Step 3 is the reduction, and known reaction. It may be carried out, for example, in an atomosphere of hydrogen, in an inert organic solvent (methanol, ethanol, tetrahydrofuran etc.) in the presence of a catalyst (palladium-carbon, palladium, platinum black, nickel etc.) at a temperature of from 0° C to 40° C.

Step 4 may be carried out by using the compound of the formula (IA-5) and a halogen compound of the general formula: R3a″-X (wherein, X represents a bromine atom or iodine atom, and $R^{3a}$ represents the same meaning as hereinbefore defined) in the presence of a base (n-butylitium etc.) in an inert organic solvent (DMSO, tetrahydrofuran etc.) at a temperature of from 0° C to 40° C.

The starting materials of the general formula (II), (III) and (IV) are on the market or may be prepared by known method.

The compounds of the general formula (IB) may be also prepared the same method as the compounds of the general formula (IA).

The reaction products may be purified by conventional methods, for example, distillation at atmospheric or reduced pressure, high performance liquid chromatography, thin layer chromatography or column chromatography using silica gel or magnesium silicate or washing or recrystallization. Purification may be carried out after each reactions or a series of reactions.

The compounds of the general formula (IA) and (IB), of the present invention, and acid addition salts thereof possess the inhibitory activity on Maillard reaction. Accordingly, the compounds of the present invention are useful for treatment and/or prevention of several diabetic complication, i.e. coronary heart disease, peripheral circulatory insufficiency or failure, cerebrovascular hindrance, neurogenous diabetes, nephropathy, arteriosclerosis, arthrosclerosis, cataracta and retinopathy, and the diseases induced by aging, i.e. atherosclerosis, senile cataract and cancer.

An inhibitory activity on Maillard reaction of the compounds of the present invention was confirmed by the screening system mentioned below.

(1) Method of experiment

In order to evaluate the inhibitory effect on Maillard reaction in vitro, of the compounds in the present invention, the measurement was carried out under conditions described below.

100 mg/ml bovine serum albumin (BSA), 200 mM glucose and 6mM test compound were dissolved in 0.5 M phosphatic buffer solution (pH 7.38), and the mixture was incubated for a week at 37° C.

After incubation, the incubated medium was diluted 100 times with the same phosphatic buffer solution, and the fluorescence spectrum was measured at the excitation maximum of 360nm and the emission maximum of 450 nm.

Inhibition percentage of the test compounds was calculated by the following equation:

$$\text{Inhibition (\%)} = \frac{\Delta I - \{\Delta I_4 - (\Delta I_1 + \Delta I_2 + \Delta I_3)\}}{\Delta I}$$

$\Delta I_1$ : fluorescence of test compounds

$\Delta I_2$ : fluorescence of (test compounds + glucose),

$\Delta I_3$ : fluorescence of (test compounds + BSA),

$\Delta I_4$ : fluorescence of (test compounds + BSA + glucose),

$\Delta I$ : fluorescence of (BSA + glucose)

(2) Results

The results are shown in the following table I.

Table I

| | Ex. No. | $-X_a-R^{1a}-R^{2a}$ or $-X_b-R^{1b}-R^{2b}$ | $R^{3a}$ or $R^{3b}$ | Salts | Name | Inhibitory effect (%) (6mM) |
|---|---|---|---|---|---|---|
| The compounds of the present invention | 1(a) | 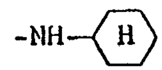 | H | - | 4-cyclohexyl semicarbozide | 100 |
| | 1(e) | 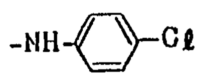 | H | hydrochloride | 4-(4-chlorophenyl) semicarbazide hydrochloride | 65 |
| | 1(f) | 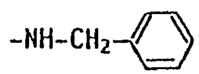 | H | hydrochloride | 4-benzylsemicarbazide hydrochloride | 90 |
| | 1(i) | $-NH(CH_2)_6NHCONHNH_2$ | H | hydrochloride | 4-[6-(3-aminoureido) hexyl]semicarbazide dihydrochloride | 100 |
| | 1(j) | 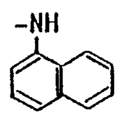 | H | hydrochloride | 4-(1-naphthyl) semicorbazide hydrochloride | 100 |
| | 1(l) | $-NH(CH_2)_7CH_3$ | H | hydrochloride | n-octylsemicarbazide hydrochloride | 100 |
| | 1(q) | 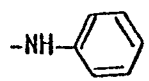 | $CH_3$ | hydrochloride | 2-methyl-4-phenylsemicarbazide hydrochloride | 74 |

Table I (continued)

| | Ex. No. | $-X_a-R^{1a}-R^{2a}$ or $-X_b-R^{1b}-R^{2b}$ | $R^{3a}$ or $R^{3b}$ | Salts | Name | Inhibitory effect (%) (6mM) |
|---|---|---|---|---|---|---|
| The compounds of the present invention | 2(a) | 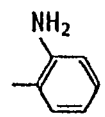 | H | hydrochloride | 2-aminobenzohydrazide | 100 |
| | 2(f) | $-(CH_2)_2-$ 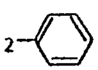 | H | - | 3-phenylpropionohydrazide | 100 |
| | 2(i) | 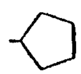 | H | - | cyclopentancarbonylhydrozine | 37 |
| | - | $-CH_3$ | H | - | acetohydrazide | 35 |
| The compared compound | | 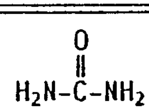 | | - | Urea | 28 |

The results in the table I show that the compounds of the present invention and acid addition salts possess an inhibitory effect on Maillard reaction and the compounds whose structures is similar to urea, possess significantly stronger acitivity than urea.

[Toxicity]

It was confirmed that the toxicity of the compounds of the present invention were very low.

According it was confirmed that the compounds of the present invention were useful for prevention and/or treatment for deseases attributed to Maillard reaction, in animals including human beings, especially human beings.

[Administration]

For the purpose above described, the compounds of the present invention of the general formula (IA), (IB) and acid addition salts thereof may normally by administered systemically or partially, usually by oral or parenteral administration.

The doses to be administered are determined depending upon age, body weight, symptom, the desired therapeutic effect, the route of administration, and the duration of the treatment etc. In the human adult, the doses per person per dose are generally between 1 mg and 1000 mg and 100 mg, by parenteral administration (preferably, intravenous administration) up to several times per day.

As mentioned above, the doses to be used depend upon various conditions. Therefore, there are cases in which doses lower than or greater than the ranges specified above may be used.

Solid compositions for oral administration in the present invention, include compressed tablets, pills, dispersible powders, and granules. In such compositions, one or more of the active compound(s) is or are, admixed with at least one inert diluent (hydroxypropylcellulose, microcrystalline cellulose, starch, polyvinylpyrrolidone, magnesium metasilicate aluminate etc.). The compositions may also comprise, as is normal practice, additional substances other than inert diluents: e.g. lubricating agents (magnesium stearate etc.), disintegrating agents (cellulose calcium gluconate etc.), stabilizing agent (lactose etc.), and assisting agent for dissolving (glutamic acid, aspertic acid etc.).

The tablets or pills may, if desired, be coated with film of gastric or enteric material (sugar, gelatin, hydroxypropylcellulose or hydroxypropylmethyl cellulose phthalate etc.), or be coated with more than two films. And further, it may be include capsules of absorbable materials such as gelatin.

Liquid compositions for oral administration include pharmaceutically-acceptable solutions, emulsions, suspensions, syrups and elixirs.

In such compositions, one or more of the active compound(s) is or are comprise in inert diluent(s) commonly used in the art (purified water, ethanol etc.).

Besides inert diluents, such compositions may also comprise adjuvants (wetting agents, suspending agents etc.), sweetening agents, flavouring agents, perfuming agents and preserving agents.

Other compositions for oral administration include spray compositions which may be prepared by known methods and which comprise one or more of the active compound(s).

Spray compositions may comprise additional substances other than inert diluents: e.g. stabilizing agents (sodium sulfite etc.), isotonic buffer (sodium chloride, sodium citrate, citric acid etc.).

For preparation of such spray compositions, for example, the method described in the United States Patnet No. 2868691 or 3095355 may be used.

Injections for parenteral administration include sterile aqueous or non-aqueous solutions, suspensions and emulsions. In such compositions, one more of active compound(s) is or are admixed at least one of inert aqueous diluent(s) (distilled water for injection, physiological salt solution etc.) or inert non-aqueous diluent(s) (propylene glycol, polyethylene glycol, olive oil, ethanol, POLYSOLBATE 80 (registered trade mark) etc.).

Injections may comprise additional other than inert diluents: e.g. preserving agents, wetting agents, emulsifying agents, dispersing agents, stabilizing agents (lactose etc.), assisting agents such as assisting agents for dissolving (glutamic acid, aspertic acid etc.).

They may be sterillized, for example, by filtration through a bacteria-retaining filter, by incorporation of sterilizing agents in the compositions or by irradiation. They also be manufactured in the form of sterile solid compositions, for example, by freeze-drying, and which can be dissolved in sterile water or some

other sterile diluents for injection immediately before used.

Other compositions for parenteral administration include liquids for external use, and endermic linimetns (ointment etc.), suppositories and pessaries which comprise one or more of the active compound(s) and may be prepared by known methods.

The following examples illustrate the compounds of the present invention and the process for the preparation of them, but not limit the present invention.

The solvents in the parentheses show the developing or eluting solvents and the rations of the solvents used are by volume in chromatographic separations. "IR" were measured by KBr tablet method.

Example 1

4-(4-nitrophenyl)semicarbozide hydrochloride

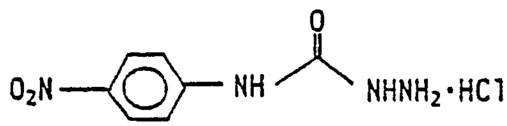

80% hydrazine hydrate (6.10 g) was suspended in ether (50 ml). A solution of 4-nitrophenyl isocyanate (2.00 g) in ether (400 ml) was added to the suspension, keeping the temperature from 8 to 12 °C. The temperature of the mixture solution was raised to a room temperature. The mixture was stirred for 30 minutes. Ether was removed by decantation. Water was added to the mixture. The mixture was stirred. The crystals precipitated was obtained by filtration. The crystals was added to hot ethanol. The insoluble material was removed by filtration. The filtrate was concentrated. The residue was recrystallized from ethyl acetate to purify. This crystals was dissolved in ethyl acetate. A mixture (4 ml) of 4N hydrochloric acid and ethyl acetate was added dropwise to the solution. White precipitate deposited was obtained by filtration. The precipitate was washed with ethyl acetate, evaporated and dried to give the title compound having the following physical data (224 mg):

TLC: Rf 0.35 (methylene chloride: methanol = 8 : 1);

IR : $\nu$ 2710, 1700, 1620, 1560, 1490, 1410, 1350, 1330, 1305, 1250, 1220, 1180, 1110, 850, 750, 690 $cm^{-1}$.

Reference example 1(a) ~ 1 (u)

The compounds of the present invention shown in the following table II were obtained with using the compound of the general formula:

$R^{2a}$-$R^{1a}$-NCO (II)

(wherein, all of the symbols represent the same meaning as hereinbefore defined.)

and the compound of the general formula:

$H_2NNH$-$R^{3a}$ (III)

(wherein, the symbol represents the same meaning as hereinbefore defined.) as the starting materials, by the same procedure as example 1, with the proviso that the compounds in example 1(a), 1(r) and 1(u) were not converted into hydrochlorides.

Table II

| Ex. No. | Structural formula | Name | TLC | IR |
|---|---|---|---|---|
| 1(a) | 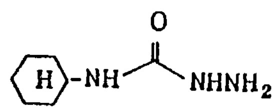 | 4-cyclohexyl semicarbazide | Rf 0.29 (methylene chloride: methanol = 8 : 1) | ν 3390,3350,3290,2925, 2850,1620,1560,1440, 1310,1280,1250,1150, 1050,885,740,655 $cm^{-1}$ |
| 1(b) | 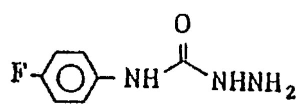 •HCℓ | 4-(4-fluorophenyl) semicarbazide hydrochloride | Rf 0.33 (methylene chloride: methanol = 8 : 1) | ν 2670,1685,1620,1565, 1510,1410,1310,1235, 835 $cm^{-1}$ |
| 1(c) | 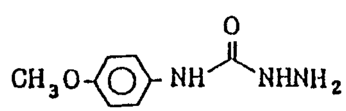 •HCℓ | 4-(4-methoxyphenyl) semicarbazide hydrochloride | Rf 0.28 (methylene chloride: methanol = 8 : 1) | ν 3270,1680,1605,1560, 1505,1410,1300,1250, 1175,1105,1030,830, 565,520 $cm^{-1}$ |
| 1(d) | 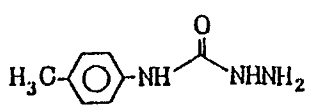 •HCℓ | 4-(4-tolyl) semicarbozide hydrochloride | Rf 0.28 (methylene chloride: methanol = 8 : 1) | ν 3280,2960,2670,1685, 1615,1555,1510,1470, 1410,1320,1300,1250, 820, 795, 765, 645, 620, 505 $cm^{-1}$ |

Table II (continued)

| Ex. No. | Structural formula | Name | TLC | IR |
|---|---|---|---|---|
| 1(e) | 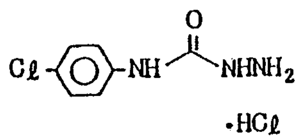 | 4-(4-chlorophenyl) semicarbazide hydrochloride | Rf 0.27 (methylene chloride: methanol = 8 : 1) | v 2660,1690,1610,1550, 1480,1405,1310,1290, 1245,1095,1015,985, 830, 790, 750, 690, 640, 620, 510 $cm^{-1}$ |
| 1(f) | 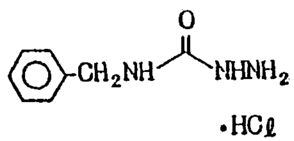 | 4-benzylsemicarbazide hydrochloride | Rf 0.25 (methylene chloride: methanol = 8 : 1) | v 2960,2675,1680,1560, 1480,1265,780, 760, 695 $cm^{-1}$ |
| 1(g) | 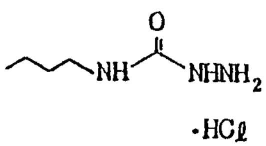 | 4-n-butylsemicarbazide hydrochloride | Rf 0.27 (methylene chloride: methanol = 8 : 1) | v 3310,2950,2675,1690, 1565,1495,1285,650 $cm^{-1}$ |
| 1(h) | 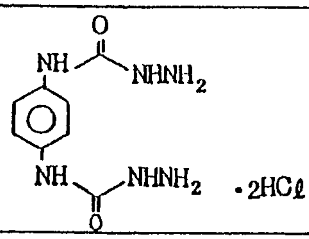 | 4-[4-(3-aminoureido) phenyl]semicarbazide hydrochloride | Rf 0.25 (acetonitrile: methanol = 3 : 1) | v 3290,3200,2980,2670, 1685,1585,1510,1480, 1415,1313,1240,980, 840, 820, 780, 620, 520 $cm^{-1}$ |

Table II (continued)

| Ex. No. | Structural formula | Name | TLC | IR |
|---|---|---|---|---|
| 1(i) | 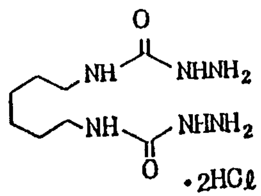 | 4-[6-(3-aminoureido) hexyl]semicarbazide | Rf 0.31 (acetonitrile: methanol = 3 : 2) | ν 3290,2930,2660, 1680,1560,1485, 1270 $cm^{-1}$ |
| 1(j) | 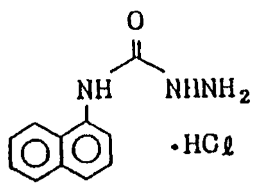 | 4-(1-naphthyl) semicarbazide hydrochloride | Rf 0.38 (methylene chloride: methanol = 8 : 1) | ν 2670,1690,1600,1555, 1400,1340,1260, 790, 770 $cm^{-1}$ |
| 1(k) | 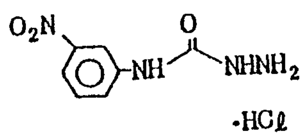 | 4-(3-nitrophenyl) semicarbazide hydrochloride | Rf 0.32 (methylene chloride: methanol = 8 : 1) | ν 2990,2680,1690,1610, 1560,1525,1480,1345,1325,1305,1255,1165, 1110,1085, 995, 895, 835, 810, 740, 625 $cm^{-1}$ |
| 1(l) | 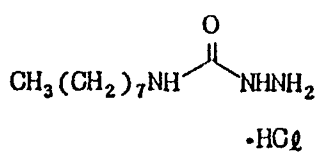 | n-octylsemicarbazide hydrochloride | Rf 0.39 (methylene chloride: methanol = 8 : 1) | ν 3300,2930,2870,2680, 1690,1575,1495,1280, 1220,1185,795, 655 $cm^{-1}$ |

Table II (continued)

| Ex. No. | Structural formula | Name | TLC | IR |
|---|---|---|---|---|
| 1(m) | 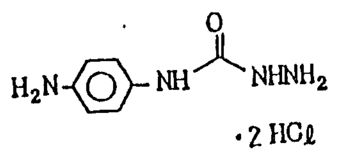 | 4-(4-aminophenyl) semicarbazide dihydrochloride | Rf 0.15 (methylene chloride: methanol = 8 : 1) | ν 3480~3420, 3270, 3050~2880, 1710, 1620, 1570, 1500, 1320, 1250, 1100, 830, 510 $cm^{-1}$ |
| 1(n) | 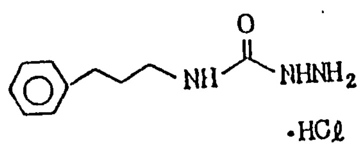 | 4-(3-phenylpropyl) semicarbozide hydrochloride | Rf 0.37 (methylene chloride: methanol = 8 : 1) | ν 3300, 3200, 3000, 2860, 2675, 1685, 1570, 1495, 1450, 1288, 740, 695, 643 $cm^{-1}$ |
| 1(o) | 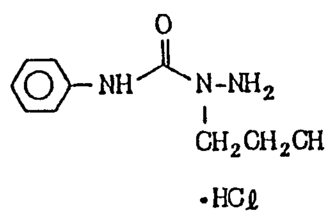 | 2-propyl-4-phenylsemi carbazide hydrochloride | Rf 0.34 (ethyl acetate: hexane = 3 : 2) | ν 2970~2890, 1680~1645, 1590, 1520, 1445, 1377, 1312, 1245, 1155, 1020, 900, 755, 690 $cm^{-1}$ |

Table II (continued)

| Ex. No. | Structural formula | Name | TLC | IR |
|---|---|---|---|---|
| 1(p) | 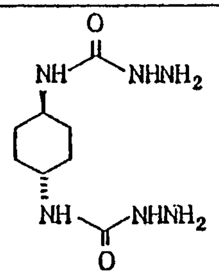 ·2HCl | trans-4-[4-(3-amino ureido)cyclohexyl] semicarbazide dihydrochloride | Rf 0.27 (acetonitrile: methanol = 3 : 2) | ν 3300,3000,2825,2660, 1675,1595,1550,1505, 1490,1325,1310,1273, 1237,1210,1170,1080, 968, 900, 800, 763, 658, 530 $cm^{-1}$ |
| 1(q) | 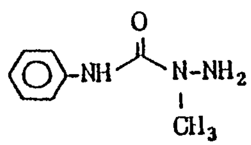 ·HCl | 2-methyl-4-phenylsemicarbazide hydrochloride | Rf 0.46 (methylene chloride: methanol = 12 : 1) | ν 3270, 2880,2750,2675, 1680,1600,1540,1440, 1355,1315,1300,1240, 1190,1025,765, 700 $cm^{-1}$ |
| 1(r) | 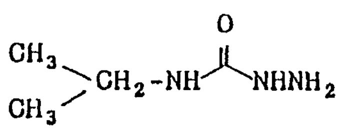 | 4-isopropylphenyl semicarbazide | Rf 0.31 (methylene chloride: methanol = 8 : 1) | ν 3320,3240,2980,1660, 1540,1460,1440,1380, 1360,1330,1280,1180, 1125,875, 790, 680, 590 $cm^{-1}$ |

Table II (continued)

| Ex. No. | Structural formula | Name | TLC | IR |
|---|---|---|---|---|
| 1(s) | 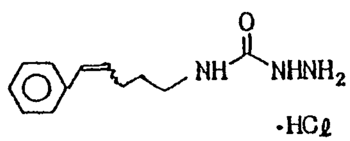 | 4-(5-phenyl-4-pentenyl)semicarbazide hydrochloride | Rf 0.38 (methylene chloride: methanol = 8 : 1) | ν 3300,3190,3020,2870, 2660,1685,1560,1490, 1440,1280,1215,1180, 965, 770, 690, 645 $cm^{-1}$ |
| 1(t) | 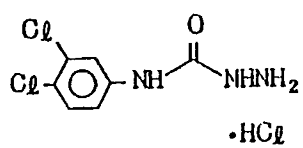 | 3,4-dichlorophenyl semicarbazide hydrochloride | Rf 0.31 (methylene chloride: methanol = 9 : 1) | ν 3175,3000~2900,1680, 1600,1540,1470,1380, 1300,1243,810 $cm^{-1}$ |
| 1(u) | 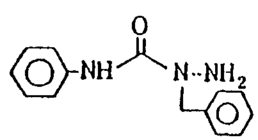 | 2-benzyl-4-phenylsemi carbazide | Rf 0.42 (ethyl acetate: hexane = 1 : 2) | ν 3375,3330,3300,3200, 1640,1590,1515,1440, 1355,1305,1200,1100, 1075,995, 975, 955, 870, 750, 730, 695, 640 $cm^{-1}$ |

Example 2

4-methoxybezohydrazide

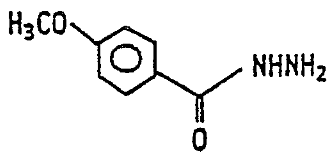

Methyl p-methoxybezoate (1.1 g, 6.6 mmol) was dissolved in ethanol (10 ml). 80% hydrazine hydrate (4 ml) was added to the solution. The reaction mixture was refluxed for 5 hours and the cooled to a room temperature. The reaction mixture was eaporated. The residue was washed with cooled water three times, dried and purified by recrystallization from ethanol to give the title compound (254 mg) having the physical data:
TLC: Rf 0.37 (chloroform : methanol = 9 : 1);
IR : $\nu$ 3300, 1590, 1470, 1310, 1240,
1160, 1020, 910, 830, 750, 590 $cm^{-1}$.

Example 2(a) ~ 2(k)

The compounds of the present invention shown in the following table III were obtained by using the compounds of the general formula:
$R^{2a}$-$R^{1a}$-$COOR^{4a}$
(wherein, $R^{4a}$ represents a methyl or ethyl group, the other symbols represent the same meaning as hereinbefore defined.) as starting materials, by the same procedure as example 2, with the proviso that the compound in example 2(a) was converted into hydrochloride by the same procedure as example 1.

Table III

| Ex. No. | Structural formula | Name | TLC | IR |
|---|---|---|---|---|
| 2(a) | 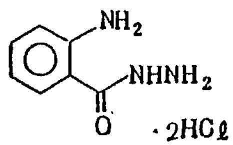 ·2HCℓ | 2-aminobenzohydrazide | Rf 0.48 (chloroform: methanol = 10 : 1) | ν 3500~2400, 3475, 3320, 1615, 1570, 1495, 1260, 950 $cm^{-1}$ |
| 2(b) | 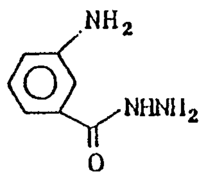 | 3-aminobenzohydrazide | Rf 0.31 (chloroform: methanol = 10 : 1) | ν 3500~2200, 1600, 1620~1570, 1510, 1480, 1330 $cm^{-1}$ |
| 2(c) | 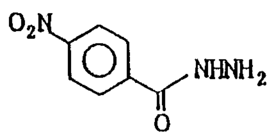 | 4-nitrobenzohydrazide | Rf 0.53 (chloroform: methanol = 9 : 1) | ν 3230, 1585, 1500, 1330, 1090, 930, 850, 700, 670, 630 $cm^{-1}$ |
| 2(d) | 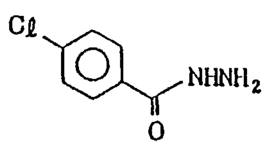 | 4-chlorobenzohydrazide | Rf 0.52 (chloroform: methanol = 9 : 1) | ν 3200, 3000, 1600, 1540, 1340, 1090, 980, 870, 830, 720, 660 $cm^{-1}$ |

Table III (continued)

| Ex. No. | Structural formula | Name | TLC | IR |
|---|---|---|---|---|
| 2(e) | 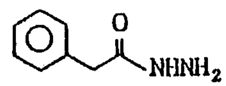 | benzylhydrazide | Rf 0.40 (chloroform: methanol = 9 : 1) | ν 3280, 1630, 1510, 1405, 1345, 1250, 990, 690 $cm^{-1}$ |
| 2(f) | 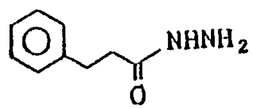 | 3-phenylpropionohydrazide | Rf 0.51 (chloroform: methanol = 9 : 1) | ν 3300, 3160, 1600, 1510, 990, 740, 690, 580, 490 $cm^{-1}$ |
| 2(g) | 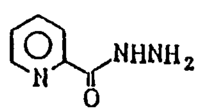 | 2-pyridinecarbonyl hydrazine | Rf 0.39 (chloroform: methanol = 10 : 1) | ν 3400~2850, 3300, 3210, 1670, 1640, 1505, 970 $cm^{-1}$ |
| 2(h) | 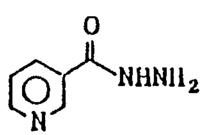 | nicotinoylhydrazine | Rf 0.18 (chloroform: methanol = 10 : 1) | ν 3400~2600, 3200, 3000, 1660, 1530, 1335, 950 $cm^{-1}$ |

Table III (continued)

| Ex. No. | Structural formula | Name | TLC | IR |
|---|---|---|---|---|
| 2(i) | 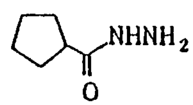 | cyclopentancarbohydrazide | Rf 0.31 (chloroform: methanol = 19 : 1) | ν 3270, 1610, 1515, 1250, 1000, 640 $cm^{-1}$ |
| 2(j) | 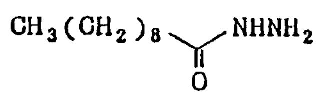 | decanoylhydrazine | Rf 0.24 (chloroform: methanol = 19 : 1) | ν 3270, 1615, 1510, 1450, 1370, 1000, 680, 610 $cm^{-1}$ |

Example 3

Styrylhydrazide

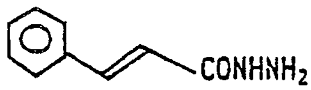

Cinnamoyl chloride (2 g) was dissolved in THF (60 ml) in an atmosphere of argon at a temperature of 0°C. 80% hydrazine hydrate was added dropwise to the solution. The mixture solution was stirred for 3 hours and then filtrated. The filtrate was evaporated. The residue was purified by column chromatography on silica gel and recrystallized from a mixture solution of methanol and benzene to give the title compound having the following physical data.
TLC: Rf 0.58 (chloroform : methanol = 3 : 1);
IR.: ν 3350 2800, 3180, 3005, 1640, 1600, 1330, 1185 $cm^{-1}$.

[Preparative example]

The following components were admixed in conventional method and punched out to obtain 100 tablets each containing 50 mg of active ingredient.

| | |
|---|---|
| ° 4-benzylsemicarbazide hydrochloride | 5 g |
| ° Cellulose Calcium gluconate (disintegrating agent) | 0.2 g |
| ° Magnesium stearate (lubricating agent) | 0.1 g |
| ° Microcrystaline cellulose | 4.7 g |

## Claims

1) A novel carbazoyl derivative of the general formula:

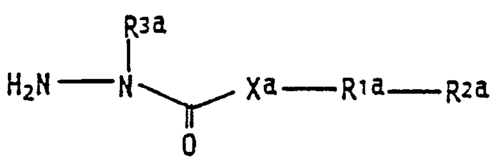

(IA)

(wherein, $R^{1a}$ represents a bond, alkylene group of from 1 to 6 carbon atom(s) or alkenylene group of from 2 to 6 carbon atoms,
$R^{2a}$ represents a carbocyclic or heterocyclic ring unsubstituted or substituted by from one to three halogen atom(s), hydroxy group(s), nitro group(s), amino group(s), alkyl or alkoxy group(s) of from 1 to 4 carbon atom(s), 3-aminoureido group, phenoxy group or acylamino group of from 2 to 5 carbon atoms, or
$R^{1a}$ together with $R^{2a}$ represents an alkyl group of from 1 to 12 carbon atom(s) unsubstituted or substituted by 3-aminoureido group,
$R^{3a}$ represents (1) a hydrogen atom,
(2) an alkyl group of from 1 to 6 carbon atom(s)
(3) a phenyl or benzyl group unsubstituted or substituted by from one to three halogen atom(s), alkyl or alkoxy group(s) of from 1 to 4 carbon atom(s), hydroxy group(s) or nitro group(s),

$X_a$ represents a bond or imino group, with the proviso that the compounds, wherein the groups shown by -Xa-$R^{1a}$-$R^{2a}$ represent methyl, phenyl, 4-aminophenyl, anilino, 2-nitrophenyl, 4-nitrophenyl, 2-chlorophenyl, 4-chlorophenyl, benzyl, 3-bromophenyl, 4-bromophenyl, 2-thienyl, 4-pyridyl or 4-hydroxyphenyl group and the groups shown by $R^{3a}$ represent a hydrogen atom, are excluded.) or an acid addition salt thereof.

2) A compound according to claim 1, wherein $X_a$ represents a bond.

3) A compound according to claim 1, wherein $X_a$ represents an imino group.

4) A compound according to claim 1, wherein $R^{1a}$ represents a bond, methylene, ethylene, trimethylene, vinylene or 1-pentenylene group.

5) A compound according to claim 1, wherein $R^{2a}$ represents a carbocyclic or heterocyclic ring substituted by a fluorine atom, chlorine atom, hydroxy gorup, methyl, methoxy, nitro, amino or 3-aminoureido group.

6)A compound according to claim 1, wherein $R^{2a}$ represents a unsubstituted carbocyclic or heterocylic ring

7) A compound according to claim 1, wherein $R^{1a}$ together with $R^{2a}$ represents an isopropyl, butyl, octyl, nonyl or 6-(3-aminoureido)hexyl group.

8) A compound according to claim 1, wherein $R^{3a}$ represents a hydrogen atom, methyl, propyl or benzyl group.

9) A compound according to claim 2, which is
3-aminobenzohydrazide
, 3-phenylpropionohydrazide or
2-pyridinecarbonylhydrazine.

10) A compound according to claim 3, which is
4-cyclohexylsemicarbazide,
4-[6-(3-aminoureido)hexyl]semicarbazide,
4-(4-aminophenyl)semicarbazide,
4-(3-phenylpropyl)semicarbazide,
4-[4-(3-aminoureido)cyclohexyl]semicarbazide or
2-methyl-4-phenylsemicarbazide.

11) A process for the preparation of the carbazoyl derivatives of the general formula:

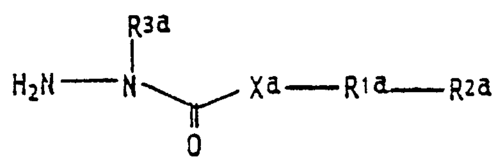
(I)

(wherein the symbols represent the same meanings as described hereinbefore.)
which is characterized by

i) reacting the compound of the general formula:

$R^{2a}$-$R^{1a}$-NCO (II)

(wherein the symbols represent the same meanings as described hereinbefore.)
and the compounds of the general formula:

$H_2N$-NH-$R^{3a}$ (III)

(wherein the symbols represent the same meanings as described hereinbefore.),

ii) reacting the ester, acid halide or mixed acid anhydride derived from the compound of the general formula:

$R^{2a}$-$R^{1a}$-COOH (IV)

(wherein the symbols represent the same meanings as described hereinbefore.)
and the compound of the general formula:

$H_2N$-NH-$R^{3a}$ (III)

(wherein the symbols represent the same meanings as described hereinbefore.)

iii) reducing the compound of the general formula:

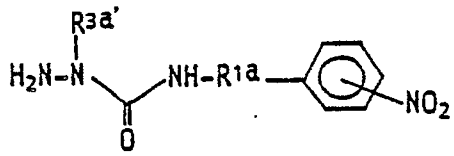

(IA-3)

(wherein, $R^{3a'}$ represents 1) a hydrogen atom, 2) an alkyl group of from 1 to 6 carbon atom(s) or 3) a phenyl or benzyl group unsubstituted or substituted by from one to three halogen atom(s), alkyl or alkoxy group of from 1 to 4 carbon atom(s) or hydroxy group, and the other symbols represent the same meaning as described hereinbefore.) or

iv) alkylating the compound of the general formula:

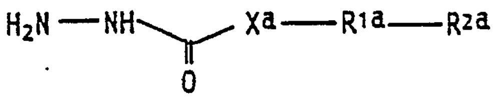

(IA-5)

(wherein, the symbols represent the same meaning as described hereinbefore.)

12) A Pharmaceutical composition, which comprises an effective amount of the carbazoyl derivative of the general formula:

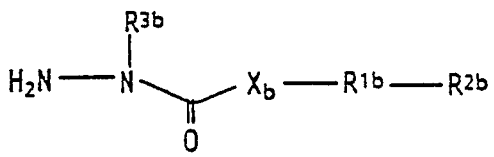

(IB)

(wherein, $R^{1b}$ represents a bond, alkylene group of from 1 to 6 carbon atom(s) or alkenylene group of from 2 to 6 carbon atoms,
$R^{2b}$ represents a carbocyclic or heterocyclic ring unsubstituted or substituted by from one to three halogen atom(s), hydroxy group, nitro group, amino group, alkyl or alkoxy group of from 1 to 4 carbon atom(s), 3-aminoureido group, phenoxy group or acylamino group of from 2 to 5 carbon atoms, or $R^{1b}$ together with $R^{2b}$, represents an alkyl group of from 1 to 12 carbon atom(s) unsubstituted or substituted by 3-aminoureido group,
$R^{3b}$ represents (1) a hydrogen atom,
(2) an alkyl group of from 1 to 6 carbon atom(s) or
(3) a phenyl or benzyl group unsubstituted or substituted by from one to three halogen atom, alkyl or alkoxy group of from 1 to 4 carbon atom(s), hydroxy group or nitro group,
$X_b$ represents a bond or imino gorup,
with the proviso that the compounds wherein the groups shown by $-X_b-R^{1b}-R^{2b}$ represent a phenyl group, 4-aminophenyl gorup, anilino group and 2-thienyl group and the groups shown by $R^{3b}$ represent a hydrogen atom, are excluded.),
or an acid addition salt thereof and pharmaceutically acceptable carrier and/or coating.

13) A pharmaceutical composition according to claim 12, which comprises an effective amount of 4-methoxybenzohydrazide.

14) The method for treatment or prevention of several diabetic complications and deseases induced by aging which comprises the administration of an effective amount of the carbazoyl derivative of the general formula (IB).

Claims for the following Contracting States: ES, GR

A process for the preparation of the carbazoyl derivatives of the general formula:

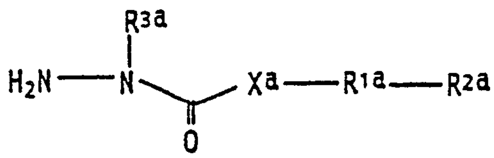

(I)

(wherein the symbols represent the same meanings as described hereinbefore.) which is characterized by

i) reacting the compound of the general formula:

$R^{2a}$-$R^{1a}$-NCO (II)

(wherein the symbols represent the same meanings as described hereinbefore.) and the compounds of the general formula:

$H_2N$-NH-$R^{3a}$ (III)

(wherein the symbols represent the same meanings as described hereinbefore.),

ii) reacting the ester, acid halide or mixed acid anhydride derived from the compound of the general formula:

$R^{2a}$-$R^{1a}$-COOH (IV)

(wherein the symbols represent the same meanings as described hereinbefore.) and the compound of the general formula:

$H_2N$-NH-$R^{3a}$ (III)

(wherein the symbols represent the same meanings as described hereinbefore.)

iii) reducing the compound of the general formula:

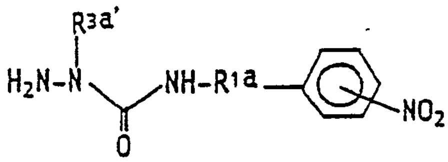

(IA-3)

(wherein, $R^{3a'}$ represents 1) a hydrogen atom, 2) an alkyl group of from 1 to 6 carbon atom(s) or 3) a phenyl or benzyl group unsubstituted or substituted by from one to three halogen atom(s), alkyl or alkoxy group of from 1 to 4 carbon atom(s) or hydroxy group, and the other symbols represent the same meaning as described hereinbefore.) or

iv) alkylating the compound of the general formula:

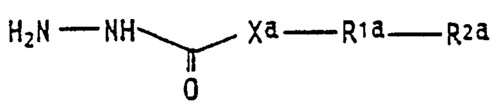

(IA-5)

(wherein, the symbols represent the same meaning as described hereinbefore.)